# NEUE EUROPÄISCHE PATENTSCHRIFT

(11) **EP 2 213 273 B2**
(45) Veröffentlichungstag und Bekanntmachung des Hinweises auf die Entscheidung über den Einspruch: **31.08.2022**
(45) Hinweis auf die Patenterteilung: 27.07.2016
(21) Anmeldenummer: 10000455.5
(22) Anmeldetag: 19.01.2010
(51) Int. Cl.: A61H 23/00, A61N 7/00, B06B 1/18, B25D 9/26, A61B 17/225

(54) **Parametereinstellung bei einem Gerät zur Druckwellenbehandlung**
Setting parameters for a device for shock wave treatment
Réglage des paramètres d'un appareil de traitement des ondes de pression

(30) Priorität: 02.02.2009 DE 102009007129
(43) Veröffentlichungstag der Anmeldung: 04.08.2010
(60) Teilanmeldung: 16001345.4
(73) Patentinhaber: STORZ MEDICAL AG, 8274 Tägerwilen (CH)
(72) Erfinder: Novak, Pavel, Dr., 8234 Stetten/SH (CH); Marlinghaus, Ernst H. Dr., 8598 Bottighofen/TG (CH); Schulz, Manfred, 8274 Tägerwilen/T (CH); Katona, Josef, 78315 Radolfzell (DE)
(74) Vertreter: Szynka, Dirk

(56) Entgegenhaltungen:
- EP-A1- 1 574 198
- EP-A1- 1 785 164
- EP-A1- 2 181 730
- DE-B3-102006 062 356
- DE-U1-202004 011 323
- US-B1- 6 575 990

## Beschreibung

Die vorliegende Erfindung betrifft ein Gerät zum Behandeln des menschlichen oder tierischen Körpers durch mechanische Druckwellen.

Solche Vorrichtungen sind an sich bereits bekannt, insbesondere aus dem Bereich der Lithotripsie. Dort werden mit fokussierten mechanischen Druckwellen Körperkonkremente, insbesondere Steine im Körpergewebe, zertrümmert. Neben der Erzeugung durch elektrische Entladungen in Wasser sind auch Geräte entwickelt worden, die mechanische Druckwellen durch das Aufeinanderprallen eines beschleunigten Schlagteils und eines Prallkörpers erzeugen und mit Hilfe des Prallkörpers in Körpergewebe einkoppeln. Solche Geräte sind sowohl in der Lithotripsie mit einem direkten Kontakt zwischen dem Prallkörper bzw. einer mit dem Prallkörper verbundenen Sonde und dem Stein als auch bei anderen Behandlungen von biologischen Körpersubstanzen eingesetzt worden. Insbesondere sind hier die Behandlung von Muskelerkrankungen und von Erkrankungen im Übergangsbereich zwischen Muskeln und Knochen zu nennen.

Ein Beispiel für ein Gerät, das zu dem zuletzt genannten Typ zu rechnen ist, ist die in der EP 0 991 447 dargestellte Vorrichtung. Bei dieser sollen unfokussierte Druckwellen in das Körpergewebe eingekoppelt werden.

Bei solchen Geräten kann die Heftigkeit der eingekoppelten Druckwelle durch Einstellung eines Druckwerts einer pneumatischen Versorgungseinrichtung verändert werden. Je höher der anstehende Pneumatikdruck, umso heftiger wird das Schlagteil beschleunigt und umso größer ist der Impuls- und Energieübertrag auf den Prallkörper.

Überdies erlauben viele Geräte die Einstellbarkeit der Wiederholfrequenzen pneumatischer Pulse und damit der Wiederholfrequenz der Schläge des Schlagteils auf den Prallkörper und der daraus resultierenden eingekoppelten Druckwellen.

Im Übrigen erlauben viele Geräte den Austausch von Prallkörpern gegen hinsichtlich Geometrie und/oder Masse abweichende andere.

Die DE 10 2006 062 356 B3 offenbart ein pneumatisch betriebenes Druckwellenbehandlungsgerät mit einem Schlagteil und einem Prallkörper. Dabei soll das Schlagteil pro Druckgaspuls mehrfach auftreffen und muss der Druckgaspuls dementsprechend ausreichend lange vorgegeben sein. Im Übrigen wird festgestellt, dass die Betriebsfrequenz naturgemäß kleiner als der Kehrwert der Druckgaspulsdauer ist.

Dieses Dokument liegt dem Oberbegriff des Anspruchs 1 und des Anspruchs 8 zugrunde.

Ferner wird auf die nicht vorveröffentlichte, aber prioritätsältere EP 2 181 730 A1 verwiesen.

Der vorliegenden Erfindung liegt auf dieser Grundlage das technische Problem zugrunde, solche Geräte hinsichtlich der Parametereinstellung weiter zu verbessern und ein entsprechendes Verfahren zum Einstellen anzugeben.

Dieses Problem wird gelöst durch ein Verfahren zum Einstellen einer Druckgasbeaufschlagungszeit eines Geräts zum Behandeln des menschlichen oder tierischen Körpers durch eine mechanische Druckwelle nach Anspruch 1 und ein entsprechend ausgelegtes Gerät nach Anspruch 8.

Bevorzugte Ausgestaltungen sind in den abhängigen Ansprüchen angegeben und werden im Folgenden erläutert, wobei sich die einzelnen Merkmale grundsätzlich sowohl auf die Verfahrenskategorie als auch auf die Vorrichtungskategorie und schließlich auch auf bevorzugte Verwendungen des Geräts richten, ohne dass hierzwischen im Einzelnen explizit unterschieden wird.

Die Offenbarung geht von der Einstellung zumindest eines Parameters aus, d. h. des Druckwerts oder der Frequenz, betrifft aber natürlich umso mehr Fälle, in denen beide Parameter verändert werden. Unter Einstellung ist hier eine vom Nutzer selbst vorzunehmende Veränderung zu verstehen, also nicht eine lediglich im Rahmen von Wartungsarbeiten mögliche Verstellung. Vielmehr sollen Mittel zum Verändern der entsprechenden Parameter durch Bedienung von Bedienelementen, Verwendung von vorhandenem oder mitgeliefertem Standardwerkzeug oder auch Anschluss eines anderen elektronischen Geräts und Nutzung desselben als Terminal zur Verstellung vorgesehen sein. Auf die Frage, aus welchen therapeutischen Gründen im Einzelfall unterschiedliche Druckwerte und/oder Frequenzwerte gewünscht sein können, wird hier nicht näher eingegangen. Vielmehr soll als Ausgangsbasis vorausgesetzt werden, dass in bestimmten Fällen und für bestimmte Indikationen unterschiedliche Parameter oder Parametersätze gewünscht sind, unter Umständen auch mit Rücksicht auf einen bestimmten aus einer zur Auswahl stehenden Mehrzahl von Prallkörpern.

Auf dieser Grundlage liegt der Erfindung die Beobachtung der Erfinder zugrunde, dass sich mit einer parameterabhängig veränderlichen Zeitdauer der Druckpulse verschiedene Vorteile erreichen lassen. So lässt sich beobachten, dass pneumatisch oder anderweitig mit Druckgas betriebene Geräte des beschriebenen Typs nur für bestimmte Parametereinstellungen optimal funktionieren und für manche Parametereinstellungen sogar Funktionsstörungen auftreten können. Konkret kann dies bedeuten, dass die "Ausbeute" bei bestimmten Einstellungen für Druck und/oder Frequenz, d. h. die Erzeugung einer bei dem bestimmten Wert möglichst kräftigen Druckwelle, in den allermeisten Fällen noch deutlich optimiert werden kann. Grundsätzlich kann es als Vorteil angesehen werden, die Druckgasversorgungseinrichtung möglichst wenig zu belasten, also einen möglichst geringen Druck und/oder eine möglichst geringe Gasmenge der Versorgung einzusetzen, um eine bestimmte Druckwellenintensität zu generieren. Eine Anpassung kann erfindungsgemäß durch Einstellung von in einem gewissen Maß optimierten Zeitdauerwerten für die Druckpulse erfolgen. Wenn nämlich durch solche Optimierungsmaßnahmen letztlich eine kleine ausgelegte Druckgasversorgungseinrichtung ausreicht, so hat dies hinsichtlich Kosten, Gewicht, Baugröße und Energieaufwand Einsparvorteile. So kann bei einer bestimmten Druckgasquelle, etwa einem Kompressor, die hinsichtlich Druck und Volumenleistung gegebene Kennlinie optimal in einen möglichst großen erreichbaren Intensitätsbereich von Druckwellen umgesetzt werden, es können also mit gegebenen Kompressoren relativ heftige Druckwellen erzeugt werden, indem die Zeitdauerwerte angepasst werden. Umgekehrt kann man bei einem bestimmten zu erreichenden Bereich von Druckwellenintensitäten durch die Erfindung mit kleineren Druckgasversorgungseinrichtungen, etwa kleineren Kompressoren, auskommen. Die Erfindung kann aber auch schon dadurch von Nutzen sein, dass in erwünschten Druckparameterbereichen und/oder Frequenzbereichen ein durchweg störungsfreier Betrieb gewährleistet werden kann, indem passende Zeitdauern gewählt werden.

Der genaue Zusammenhang zwischen den Druckwerten, den Frequenzwerten und den Zeitdauerwerten der Druckpulse ist komplex und stark geräteabhängig. Bei zu kurzen Zeitdauern (insbesondere bei hohem Druck) kann wegen der endlichen Schaltzeiten beteiligter Ventile und wegen des durch Strömungswiderstände verzögerten Druckaufbaus der eigentlich verfügbare Druck u. U. nicht mehr vollständig oder nur für eine noch deutlich kürzere Zeit in einem eigentlich für die Schlagteilbeschleunigung wirksamen Bereich aufgebaut werden. Fällt der Druck infolge einer kurzen Zeitdauer relativ früh wieder ab, ist das Schlagteil möglicherweise noch nicht über einen längeren Teil seiner eigentlich vorgesehenen Beschleunigungsstrecke bewegt worden, kann gemessen an der eigentlich möglichen Beschleunigung nur unzureichend beschleunigt werden und wird bei zu frühem Zusammenbrechen der Druckfront u. U. sogar vor dem eigentlichen Aufprall auf den Prallkörper abgebremst.

Andererseits können sich durch zu lange Pulszeiten Schwierigkeiten dahingehend ergeben, dass der beschleunigende Druckpuls noch nicht hinreichend abgebaut ist, wenn das Schlagteil wieder in die Ausgangslage zurückbewegt werden soll. Wenn noch ein Restdruck ansteht, kann das dazu führen, dass das Schlagteil noch einmal Richtung Prallkörper beschleunigt wird, bevor es seine Ausgangslage eingenommen hat und damit möglicherweise unerwünschte abgeschwächte "Nachstöße" und zusätzliche schwächere Wellen ausgelöst werden. Hierzu kann erläuternd verwiesen werden auf die DE 20 2004 011 323, gemäß der dieser Effekt bewusst ausgenutzt wird, um eine Mehrzahl Schläge pro Auslösung eines Druckpulses zu erzeugen. Oft ist dies aber nicht erwünscht. Der Effekt steigt mit dem Druck an, sodass zu lange Pulsdauern mit zunehmendem Druck immer kritischer werden können.

Schließlich können längere Pulsdauern, d. h. in der Regel längere Ventilöffnungszeiten, ab einem bestimmten Wert nichts weiter zur Ausnutzung des anstehenden Versorgungsdrucks beitragen, weil das beschleunigte Schlagteil ab einer gewissen Geschwindigkeit der dann nicht mehr so schnell wie zu Anfang des Druckpulses nachströmenden Druckgasmenge gewissermaßen davonfliegt und damit von deren Entwicklung und Restaufbau nicht mehr wesentlich abhängt.

Hinsichtlich der Frequenzeinflüsse ist darauf zu achten, dass das Schlagteil bei höheren Frequenzen vor Auslösung eines neuen Beschleunigungspulses vollständig in die Ausgangslage zurückgekehrt sein sollte. Anderenfalls wird beim nächsten Beschleunigungsvorgang nicht die gesamte Beschleunigungsstrecke zur Verfügung stehen. Gleichermaßen muss der Prallkörper wieder vollständig oder zumindest weitgehend in Ruhestellung sein, um einen reproduzierbaren neuen Druckwellenprozess zu ermöglichen. Überdies kann es auch zu technischen Schwierigkeiten kommen, wenn das Schlagteil zuvor auftrifft, worauf weiter unten noch eingegangen wird.

Der grundsätzliche Zusammenhang zwischen den Druckwerten und optimalen Pulsdauerwerten wird also im Wesentlichen dadurch bestimmt, dass bei niedrigem Druck eine längere Zeit nötig ist, um genügend Luft zur Beschleunigung zuzuführen, und bei höheren Druckwerten kürzere Zeiten gewählt werden können und müssen. Zusätzlich müssen die Zeitwerte bei höheren Frequenzen abgesenkt werden, um Schwierigkeiten zu vermeiden. Im Unterschied zu konventionellen Lösungen mit fest vorgegebenen Zeitwerten können aber bei niedrigeren Frequenzen und Druckwerten deutlich längere Zeiten gewählt werden, sodass die Ausbeute gesteigert werden kann.

Die oben kurz zusammengefassten Vorgänge sind nicht nur von den beteiligten Massen und Eigenschaften von verwendeten Schaltventilen abhängig, sondern insbesondere auch vom geometrischen Aufbau des Geräts, insbesondere der von dem Druckgas durchströmten Leitungen (insbesondere der Länge und dem Durchmesser des Führungsrohres, sowie der Geometrie und dem Gewicht des Schlagteils, vgl. Ausführungsbeispiel). Die Angabe analytischer Zusammenhänge ist vor allem wegen der regelmäßig turbulenten Strömungsverhältnisse praktisch ausgeschlossen. Andererseits lassen sich optimale oder geeignete Zeitdauerwerte für jeweilige Druckund/oder Frequenzwerte ohne weiteres empirisch ermitteln. Eine begrenzte Genauigkeit ist dabei durchaus tragbar. Die Erfindung richtet sich also nicht zwingend auf eine Optimierung der Parameter im strengen Sinn, sondern kann bereits mit einer angenäherten Einstellung der Werte sinnvoll verwendet werden. Insbesondere sind stufenweise ermittelte Parametersätze ohne weiteres verwendbar, wozu auf das Ausführungsbeispiel verwiesen wird.

Die Intensität der Druckwelle kann ermittelt werden, indem beispielsweise mit optischen Verfahren (Interferometrie) die Auslenkung des Prallkörpers gemessen wird, indem piezosensorisch die Heftigkeit des durch den Prallkörper ausgeübten Stoßes ermittelt wird, indem Druckverläufe in einem an dem Prallkörper angekoppelten Wassertank gemessen werden oder in anderer Weise. Die hier beschriebenen Druckwerte beziehen sich genauso wie die Zeitdauerwerte der Druckgaspulse normalerweise auf die Versorgungsseite, also auf die beispielsweise von einer Druckgasflasche oder einem Kompressor zur Verfügung gestellten Drücke ohne Berücksichtigung der Leitungsverluste bis zum eigentlichen Ort der Schlagteilbeschleunigung bzw. auf die Ventilschaltzeiten, aus denen sich die Druckgaspulse ergeben. Die Frequenzen entsprechen dann analog den Frequenzen der Ventilbetätigung. In dieser Weise sind Druckwerte und Frequenzen bei am Markt befindlichen Geräten einstellbar und können die Zeitdauerwerte am einfachsten eingestellt werden. Es versteht sich von selbst, dass die Erfindung aber nicht wesentlich von der Frage abhängt, auf welchen genauen Messpunkt, auf welches Messverfahren und auf welche Möglichkeit der Einstellung der jeweiligen Parameter im Einzelfall zurückgegriffen wird.

Erfindungsgemäss erfolgt die Einstellung der Zeitdauerwerte automatisch, wird also von dem Gerät selbsttätig durchgeführt, wenn ein Druckwert verändert worden ist. Die Einstellung der Zeitdauerwerte kann auch durch manuelle Nachstellungen entsprechend vorgegebenen Zeitdauerwerten für bestimmte Druck- und/oder Frequenzwerte erfolgen, die in vom Gerätehersteller gelieferten Unterlagen oder selbst ermittelten Tabellen nachgeschlagen oder am Gerät an einer Anzeige abgelesen werden.

Jedenfalls ist die Auswahl nach einer im Gerät gespeicherten Tabelle vorgesehen.

Die in dem bereits zitierten Gebrauchsmuster DE 20 2004 011 323 beschriebene Einstellung im Hinblick auf eine Mehrzahl Schläge pro Puls wird im Rahmen dieser Erfindung vorzugsweise nicht durchgeführt. Sie ist aber nicht zwingend ausgeschlossen; beispielsweise lassen sich auch bei einem Doppelschlag pro Puls durchaus noch Optimierungen durch entsprechende Zeitdauereinstellungen durchführen.

Der gerade zitierte Stand der Technik veranschaulicht eine bevorzugte Bauform des Geräts, bei dem das Schlagteil entlang seiner Bewegungsstrecke nicht nur von Druckgas angetrieben wird, sondern andererseits auch Gas vor sich her treibt Dieses durch die Schlagteilbewegung unter Druck gesetzte Gas wird nicht abgelassen, sondern in einer Gegendruckkammer aufgefangen. Wenn dann der Beschleunigungsvorgang beendet ist, kann der in dieser Gegendruckkammer bestehende Gasdruck den auf der anderen Seite des Schlagteils herrschenden Druck übersteigen und für eine Rückführung des Schlagteils in die Ausgangsposition sorgen.

Die Erfinder haben festgestellt, dass bei vielen Geräten die Druckabhängigkeit der einzustellenden Zeitdauer deutlich ausgeprägter ist als die Frequenzabhängigkeit. Es ist daher bevorzugt, zumindest die Druckabhängigkeit zu berücksichtigen. Darüber hinaus ist es aber auch bevorzugt, die Frequenzabhängigkeit zur Vereinfachung einer zweidimensionalen Abhängigkeit der Zeitdauerwerte und damit deutlich erhöhten Komplexität zu vernachlässigen, sich also auf die Druckabhängigkeit zu konzentrieren. Die Erfindung betrifft übrigens auch Geräte, bei denen Einzelpulse verwendet werden (können). Dabei sind Einstellbereiche im Bereich von z. B. 0.5 bar -10 bar oder auch kleinere Bereiche sinnvoll. Typische Frequenzen können im Bereich von 0 Hz - 50 Hz liegen, wobei auch hier kleinere Einstellbereiche genügen können. Typische passende Zeiten können im Bereich zwischen 2 m/s und 25 m/s liegen, insbesondere zwischen 5 m/s und 15 m/s.

Von besonderem Vorteil ist die Erfindung bei Gerätekonstruktionen, in denen der Prallkörper elastisch gelagert ist, wozu z. B. ein und vorzugsweise zwei Elastomerringe verwendet werden können. Es wurde bereits angesprochen, dass die Sicherstellung einer ausreichenden Rückbewegung des Prallkörpers, der sich im Vergleich zu dem Schlagteil oft sehr viel langsamer bewegt, im Zusammenhang mit der Erfindung von Bedeutung sein kann. Daher hat die Erfindung eine besonders vorteilhafte Wirkung bei Prallkörpern, die sich entsprechend bewegen können, insbesondere bei solchen, die relativ große Auslenkungen zeigen. Dies betrifft insbesondere Auslenkungswerte in einem Bereich von mehr als 0,5 mm, insbesondere mehr als 1 mm.

Die Auslenkungswerte sind dabei im Sinn einer Messung bei beispielsweise an einem Stativ festgelegtem Gerät relativ zu diesem zu verstehen.

Ein weiterer Umstand, der zu einem besonderen Nutzen der Erfindung führt, liegt in einer Fangvorrichtung zum Abfangen des Schlagteils. Solche Fangvorrichtungen sind bereits dafür eingesetzt worden, ein unkontrolliertes Herausschießen des versehentlich ausgelösten Schlagteils bei abgenommenem Prallkörper zu verhindern. Es kann sich dabei beispielsweise um eine Verjüngung eines Rohrstücks handeln, in dem das Schlagteil beschleunigt werden kann. Wenn etwa das Schlagteil durch eine infolge falsch eingestellter Zeitwerte verzögerte Rückkehr noch in der Rückbewegung von dem nächsten Beschleunigungsdruckpuls erreicht und damit früher als sinnvoll wieder beschleunigt wird, kann der Prallkörper noch ausgelenkt sein und damit das Schlagteil in der Fangvorrichtung hängenbleiben. Das kann auch dann vorkommen, wenn die Entlüftung zu spät und zu langsam erfolgt. Die "Abluft" wird von dem zurückfliegenden Schlagteil derart komprimiert, dass es zu einer Umkehrung der Schlagteilbewegung und einem zweiten Aufschlag kommt, bevor der nächste Druckpuls ausgelöst wird.

Im Folgenden wird die Erfindung anhand von Ausführungsbeispielen näher erläutert, die sich auf alle Anspruchskategorien beziehen und deren einzelne Merkmale auch in anderen Kombinationen wesentlich sein können.
- Figur 1: zeigt ein erfindungsgemäßes Gerät im Längsschnitt mit schematisch dargestelltem pneumatischen Antrieb.
- Figur 2: zeigt ein zweites Ausführungsbeispiel, wobei nur ein Teil eines Handstücks im Schnitt dargestellt ist.
- Figur 3: zeigt eine Variante zu Figur 2 als drittes Ausführungsbeispiel.

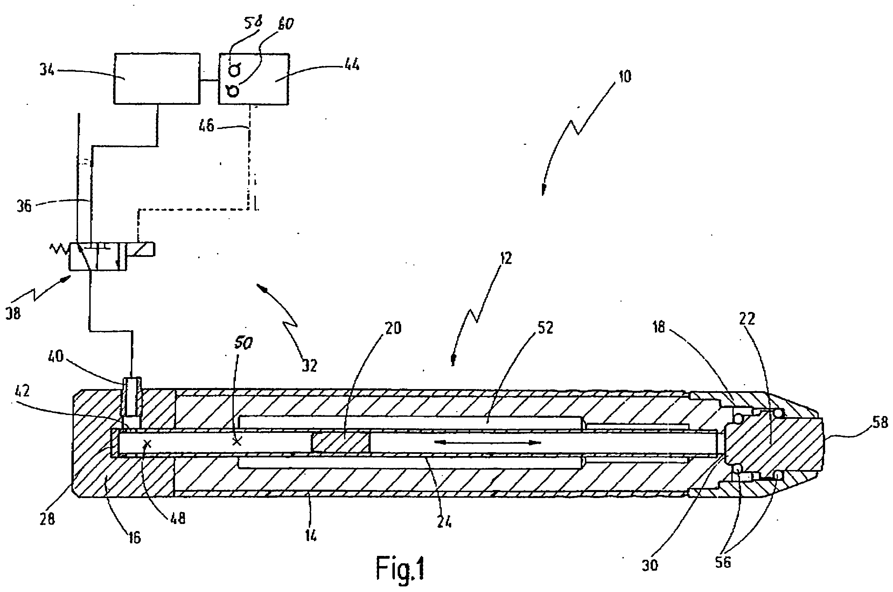

Figur 1 zeigt ein insgesamt mit 10 bezeichnetes medizinisches Gerät zur Behandlung des menschlichen Körpers mit mechanischen Druckwellen, und zwar in diesem Fall zur Weichgewebebehandlung im Rahmen einer Schmerztherapie. Das Gerät besteht aus einem Handstück 12 und einer weiter unten näher erläuterten pneumatischen Druckgasversorgungseinrichtung 32. Ein behandelnder Arzt beispielsweise kann das Handstück 12 greifen und auf eine geeignete Hautpartie mit dem in Figur 1 rechten Ende aufsetzen, wobei das Handstück 12 ungefähr senkrecht auf der Haut steht.

Ein Gehäuse 14 ist mit einer proximalen Endkappe 16 und distalen Endkappe 18 versehen, die jeweils abnehmbar ausgeführt sind. In dem Gehäuse ist ein Führungsrohr 24 gehalten und dabei axial und konzentrisch angeordnet. In dem Führungsrohr ist ein Schlagteil 20 geführt, dessen Bewegungsstrecke entlang des Innenraums des Führungsrohrs 24 auf der rechten Seite durch einen Prallkörper 22 begrenzt ist, und zwar durch dessen proximale Seite 30. Diese bildet einen distalen Anschlag des Schlagteils 20, wobei der proximale Anschlag des Schlagteils 20 mit 28 bezeichnet ist und einen einfachen Abschluss des Führungsrohres 24 bildet. Dieser Abschluss ist magnetisch, sodass das Schlagteil 20 mit einer gewissen Haltekraft dort fixiert sein kann. Typischerweise beträgt die Länge des Führungsrohres 24 etwa 5 cm - 20 cm, wobei das hier praktisch maßstäblich gezeichnete Ausführungsbeispiel eine Führungsrohrlänge von 17,4 cm hat.

Der pneumatische Antrieb 32 verkörpert die Druckgasversorgungseinrichtung und weist einen üblichen pneumatischen Kompressor 34 (oder eine Druckgasflasche) auf, wobei der Kompressor 34 einen typischen Arbeitsbereich bis etwa 10 bar abdeckt. Über eine Druckleitung 36 und ein Schaltventil 38 wird ein Druckgasanschluss 40 des Handstücks 12 versorgt, der mit dem Führungsrohr 24 über eine Öffnung 42 darin kommuniziert. Bei dem Schaltventil 38 kann es sich um ein Magnetventil handeln. Eine Steuerung 44 ist mit diesem über eine Steuerleitung 46 verbunden, die gestrichelt dargestellt ist. Die Steuerung 44 kann mit dem Kompressor 34 als Baueinheit ausgeführt sein und damit ein Basisgerät zur Versorgung des Handstücks 12 bilden, wobei an letzterem günstigerweise das Schaltventil 38 angebracht ist. Das hat den Vorteil, dass das von dem Druckpuls zu befüllende Volumen klein wird. Dadurch werden stärkere und schnellere Impulse realisierbar. Dementsprechend sind die Steuerung 44 und der Kompressor 34 in Figur 1 durch eine Leitung verbunden. Basisgerät und Handstück 12 sind dann über eine die Pneumatikleitung 36 und die Steuerleitung 46 zusammenfassende Versorgungsleitung verbunden.

An der Steuerung 44 sind zwei Einstellknöpfe 58 und 60 vorgesehen, mit denen der über die Leitung 36 zur Verfügung gestellte maximale Versorgungsdruck und die Arbeitsfrequenz des Schaltventils 38 eingestellt werden können. Der Einstellknopf 58 dient zur Einstellung des Druckwerts des Kompressors 34 mittels der zwischen der Steuerung 44 und dem Kompressor 34 eingezeichneten Leitung. (Wäre die Steuerung 44 ebenfalls baulich mit dem Handstück 12 kombiniert, könnte der Einstellknopf 58 an dem Kompressor 34 selbst vorgesehen sein oder aber eine entsprechende Steuerleitung von dem Handstück 12 zu dem Kompressor 34 führen.) Die Steuerung 44 ist dabei so ausgelegt, dass sie das Schaltventil 38 mit der an dem Einstellknopf 60 eingestellten Frequenz, die in einem Bereich von 0 Hz - 50 Hz liegt ansteuert und dabei jeweils Öffnungszeiten des Ventils einstellt, die automatisch abhängig von dem an dem Einstellknopf 58 eingestellten Druckwert gewählt werden. Dabei folgt die Steuerung 44 einer abgespeicherten Look-Up-Tabelle mit beispielsweise folgenden Werten:

| | | | | | | | | | |
|---|---|---|---|---|---|---|---|---|---|
| Druckwert in bar | bis 1,0 | 1,0 - 1,2 | 1,2-1,6 | 1,6-1,8 | 1.8-2,2 | 2,2-2,8 | 2,8-3,4 | 3,4-4,2 | ab 4,2 |
| Zeitdauer in ms | 14 | 13 | 12 | 11 | 10 | 9 | 8 | 7 | 6 |

Bei diesem Ausführungsbeispiel wird zugunsten einer einfachen Ausführung auf eine Änderung der Schaltzeiten abhängig von der Frequenz verzichtet. Bei einer komplexeren Ausführung wäre die o. a. Tabelle als zweidimensionale Matrix ausgeführt, würden also die Schaltzeiten in bestimmten jeweiligen Bereichen von dem eingestellten Druckwert und dem eingestellten Frequenzwert abhängen. Als prinzipielle Regel gilt, dass mit steigendem Druck und steigender Frequenz die Druckpulse kürzer sein müssen.

Ausgehend von einem Ruhezustand des Geräts 10, also beim Betriebsstart, wird das geschlossene Schaltventil 38 durch die Steuerung 44 geöffnet. Der in Figur 1 dargestellte Zustand, in dem das Führungsrohr 24 mit der Außenatmosphäre verbunden ist, wird damit zu einem Zustand geändert, der mit dem rechten Kästchen des Ventilsymbols dargestellt ist, wobei der Versorgungsdruck über den Anschluss 40 an das Führungsrohr 24 angelegt wird. Dabei befindet sich das Schlagteil 20 zunächst in seiner Ausgangsstellung, die in Figur 1 mit 48 angedeutet ist. Der aufgebaute Druck beschleunigt das Schlagteil 20 in Richtung des Prallkörpers, wird aber noch vor dem Aufprall durch Wiederzurückschalten des Schaltventils 38 und damit Belüften des "hinter" dem Schlagteil 20 befindlichen Volumens in dem Führungsrohr 24 abgebaut. Das Schlagteil 20 trifft ungebremst auf den Prallkörper 22, dessen distale (leicht konvexe) Abschlussfläche 58 auf der Haut des Patienten aufliegt und eine mechanische Druckwelle in den Körper überträgt. Dabei führt der Prallkörper 22 infolge seiner elastischen Aufhängung in den beiden Elastomer-O-Ringen 56 eine axiale Bewegung aus.

Unmittelbar nach dem Aufprall bewegt sich das Schlagteil 20 zurück. Dabei hilft eine Gegendruckkammer 52, die mit dem Führungsrohr 24, und zwar seinem distalen Ende kurz vor der proximalen Seite 30 des Prallkörpers 22, in hier nicht näher dargestellter Weise verbunden ist. In dieser Gegendruckkammer entsteht durch die Verdrängung infolge der Bewegung des Schlagteils 20 ein das Schlagteil 20 nach dem Aufprall bis zu dem proximalen Anschlag, also dem magnetischen Abschlussstück 28, zurückführender Gegendruck. Dieser Vorgang soll nicht wesentlich durch einen verbleibenden Restdruck infolge zu späten Umschaltens des Schaltventils 38 behindert werden, wobei etwa nur die Position 50 statt der optimalen Position 48 erreicht werden würde. Andererseits soll bei dem gegebenen eingestellten Versorgungsdruck jeweils eine maximale Intensität der erzeugten Druckwelle erreicht werden, sodass bestimmte Behandlungsergebnisse mit vergleichsweise kleinen Druckwerten bzw. einem geringeren Gasverbrauch erzielt werden können. Damit kann insgesamt die Druckgasversorgung 32, insbesondere der Kompressor 34, klein ausgelegt werden.

Nach einer bestimmten Zeit wird das Schaltventil 38 wieder umgeschaltet, sodass einer neuer Auslösevorgang beginnt. Diese bestimmte Zeit ergibt zusammen mit der Einschaltzeit des Schaltventils 38 den Kehrwert der eingestellten Frequenz. Die Steuerung 44 ist dabei so ausgelegt, dass auch bei höheren einstellbaren Frequenzen keine Komplikationen infolge zu langer Schaltzeiten auftreten. Im Prinzip könnte dies bei relativ hohen Frequenzen bedeuten, dass die Schlagintensität bei gleichbleibendem eingestellten Druckwert auch frequenzabhängig wird, weil mit Rücksicht auf die Frequenz die Schaltzeiten verkürzt werden müssen. Im Bedarfsfall kann dem mit einer automatischen Erhöhung des Drucks entgegengewirkt werden, sodass die Einstellung des Drucks tatsächlich eine Einstellung der Intensität bedeutet und dabei die Schlagintensität aber frequenzunabhängig bleibt.

Typische Aufprallgeschwindigkeiten des Schlagteils liegen im Bereich von 5 m/s - 60 m/s.

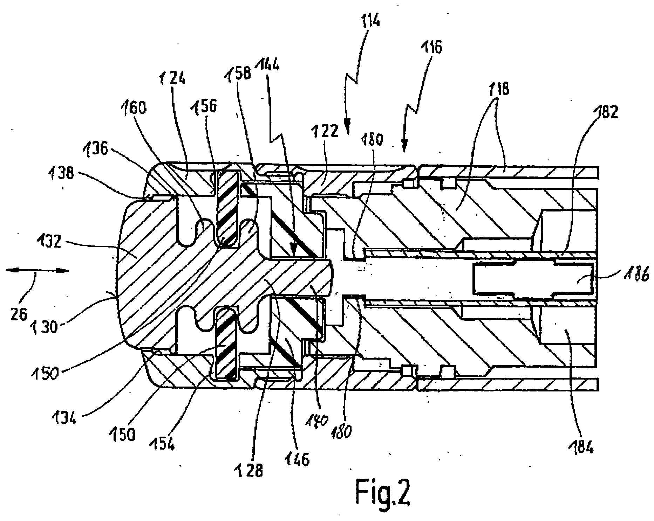

Das zweite Ausführungsbeispiel in Figur 2 ist dort nur ausschnittsweise und im Schnitt dargestellt, nämlich als alternative Ausführung des distalen Teils des Handstücks.

Das Handstück weist am distalen Ende (das hier nach links und nicht wie in Figur 1 nach rechts weist) einen Instrumentenaufsatz 114 auf, wobei ein proximales Gehäuseteil 118 zur abnehmbaren Verbindung mit einem Gehäuse des Handstücks dient. Zwei Hülsen 122 und 124 sind mit dem Gehäuseteil 118 bzw. mit der anderen Hülse verschraubt und somit abnehmbar. Der Prallkörper ist hier mit 128 beziffert und hat eine von dem ersten Ausführungsbeispiel deutlich abweichende Form, die allerdings wieder rotationssymmetrisch ist bzgl. der Achse des Handstücks. Im Übrigen entspricht die auf die Haut aufzulegende Fläche 130 weitgehend der distalen Fläche 58 im ersten Ausführungsbeispiel. Sie ist an einem Kopf 132 des Prallkörpers 128 ausgebildet, der in einer Öffnung 134 des Gehäuses 116 frei und axial längs verschieblich gehalten ist. Dabei ist der für den Fall eines Bruchs über Bunde 136 und 138 des Prallkörpers bzw. an der Öffnung 134 gesichert und mittels eines Schaftes 140 in einem PEEK- oder PTFE-Gleitlager 144 in einer Buchse 146 und ferner in einem Elastomerscheibenring 150 gelagert. Der Elastomerring liegt dabei in einer Tasche 154 in axialer Richtung im Wesentlichen nur an einer Schulter 156 an und ist an dem Schaft 140 durch Ringflansche 158, 160 gehalten. Ein geeignetes Material für den Scheibenring 150 kann Silikongummi oder Nitrilkautschuk (NBR) sein. Jedenfalls dient er der elastischen Abfederung axialer reziprozierender Bewegungen des Prallkörpers 128.

Dabei ist der gesamte Instrumentenaufsatz 114 nicht nur abnehmbar, sondern in die beschriebenen Teile zerlegbar, sodass diese einzeln ausgetauscht werden können.

Der ballistische Mechanismus zur Erzeugung der Druckwellen in dem und mittels des Prallkörpers 128 entspricht den Erläuterungen zum ersten Ausführungsbeispiel. Insbesondere ist das Führungsrohr hier mit 182 bezeichnet, die Gegendruckkammer mit 184 und das Schlagteil mit 186, das hier eine Form mit verju̅ngten Enden hat, auf die unten noch Bezug genommen wird. Bei diesem Ausführungsbeispiel kommt abgesehen von dem komplexeren Aufbau eine axial weichere Lagerung des Prallkörpers 128 hinzu, sodass die typischen Hubstrecken der Bewegung des Prallkörpers 128 deutlich größer ausfallen. Sie liegen typischerweise über 1 mm. Der Prallkörper 128 kann genauso wie der Prallkörper 22 beim ersten Ausführungsbeispiel aus Metallen, etwa Stahl oder Edelstahl, vorzugsweise gehärtet, aus Hartkunststoffen oder auch aus geeigneter Keramik bestehen.

Da sich der Prallkörper 128 hier über eine größere Strecke und in einer weicheren Lagerung bewegt, ist seine Gesamtbewegung bis zur Rückkehr in die gezeichnete Ausgangslage insgesamt zeitlich länger als beim ersten Ausführungsbeispiel. Damit besteht stärker als dort die Gefahr, dass der Prallkörper 128 noch nicht vollständig zurückgekehrt ist, wenn das Schlagteil 186 erneut heranbewegt wird. Damit könnte das Schlagteil 186 weit in die distale Rohröffnung eindringen und dort mit seinem radial stärkeren Mittenabschnitt in einer nur angedeuteten geringfügigen Innendurchmesserverringerung 180 direkt anschließend an das distale Ende des Rohres 182 steckenbleiben. Diese Fangvorrichtung 180 ist aus Sicherheitsgründen vorgesehen, um ein Herausfliegen des Schlagteils bei abgenommenen Instrumentenaufsatz 114 zu verhindern. Mit einem Einschlagen des Schlagteils in die Fangvorrichtung 180 wären Schäden verbunden, sodass der jedenfalls im Rahmen dieses Ausführungsbeispiels unerwünschte Betriebszustand eines Doppelschlags des Schlagteils 186 bei nur einem Druckpuls in diesem Zusammenhang zu erheblichen Problemen führen kann. Hier muss also besonders darauf geachtet werden, dass das Schlagteil 186 nicht schon auf einem Teil seines Rückwegs durch ein noch verbleibendes Druckgaskissen ein weiteres Mal Richtung Prallkörper 128 beschleunigt wird. Prinzipiell könnte sich ein vergleichbares Problem auch bei der Einstellung besonders hoher Arbeitsfrequenzen ergeben, wenn also der nächste reguläre Druckgaspuls entsprechend früh kommt. Dies wäre aber eine Frage der im Verhältnis zur Prallkörperdynamik zu hohen Arbeitsfrequenz und ist eher ein theoretischer Grenzfall.

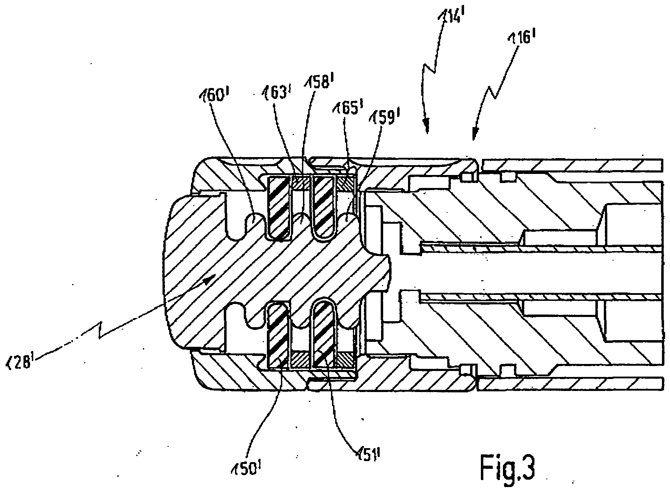

Figur 3 zeigt eine Variante zu Figur 2, wobei die entsprechenden Bezugsziffem mit einem zusätzlichen Strich versehen sind. Anstelle des erwähnten Gleitlagers 144 ist hier infolge eines zweiten elastischen Scheibenrings 151' eine doppelte Abstützung schon in der elastischen Aufhängung gegeben. Entsprechend ist ein zusätzlicher Ringflansch 159' vorgesehen. Zwischen den Scheibenringen 150' und 151' ist ein Abstandring 163' vorgesehen, wobei die Anordnung über einen Klemmring 165' festgehalten wird.

Im Übrigen gelten die vorstehenden Erläuterungen.

## Patentansprüche

1. Verfahren zum Einstellen einer Druckgasbeaufschlagungszeit eines Geräts (10) zum Behandeln des menschlichen oder tierischen Körpers durch eine mechanische Druckwelle,
welches Gerät (10) aufweist:
eine Druckgasversorgungseinrichtung (32) zur Erzeugung von mit einer Frequenz wiederholten Gasdruckpulsen,
ein Schlagteil (20, 128, 128'), das durch einen Druckgaspuls der Druckgasversorgungseinrichtung (32) beschleunigt werden kann,
einen Prallkörper (22) der von dem beschleunigten Schlagteil (20, 128, 128') getroffen werden und dabei von diesem einen Impuls zur Erzeugung der Druckwelle übernehmen kann, und eine Einrichtung (44, 58, 60) zum Einstellen eines Druckwertes der Druckgaspulse;
**dadurch gekennzeichnet, dass** das Gerät (10)
eine Einrichtung (44) zum automatischen Auswählen und Einstellen der Zeitdauer der Druckgaspulse aufweist und
bei dem Einstellverfahren vorgegebene Zeitdauerwerte für jeweilige Druckwerte entsprechend den eingestellten Druckwerten durch die Einrichtung (44) zum automatischen Auswählen und Einstellen der Zeitdauer der Druckgaspulse automatisch aus einer in dem Gerät (10) gespeicherten Tabelle ausgewählt und eingestellt werden.

2. Verfahren nach Anspruch 1, bei dem die Zeitdauerwerte so eingestellt werden, dass pro Druckgaspuls nur je ein Auftreffen des Schlagteils (20, 128, 128') auf den Prallkörper (22) erfolgt.

3. Verfahren nach einem der vorstehenden Ansprüche, bei dem das Gerät (10) so aufgebaut ist, dass das Schlagteil (20, 128, 128') entlang einer Strecke seiner Beschleunigung Gas vor sich her verdrängt und dabei in eine Gegendruckkammer (52, 184) drückt, in der sich dadurch ein Gegendruck aufbauen kann, der das Schlagteil (20, 128, 128') entlang der Strecke zurück bewegen kann.

4. Verfahren nach einem der vorstehenden Ansprüche, bei der die Zeitdauerwerte ausschließlich entsprechend den eingestellten Druckwerten ausgewählt und eingestellt werden.

5. Verfahren nach einem der vorstehenden Ansprüche, bei dem der Prallkörper elastisch gelagert ist, insbesondere über zumindest einen Elastomerring (56, 150, 150', 151'), sodass er durch die Impulsübernahme von dem Schlagteil (20, 128, 128') ausgelenkt werden kann.

6. Verfahren nach Anspruch 5, bei dem das Gerät (10) so ausgelegt ist, dass sich Auslenkungswerte in einem Bereich von 0,5 mm bis 5 mm ergeben.

7. Verfahren nach einem der vorstehenden Ansprüche, bei dem das Gerät (10, 44) eine Fangvorrichtung (180) zum Abfangen des Schlagteils bei Auslösen seiner Beschleunigung bei abgenommenen Prallkörper aufweist, insbesondere in Form einer Verjüngung (180) an einem prallkörperseitigen Ende eines zur Beschleunigung des Schlagteils dienenden Rohrstücks (182).

8. Gerät (10) zum Behandeln des menschlichen oder tierischen Körpers durch eine mechanische Druckwelle mit:
einer Druckgasversorgungseinrichtung (32) zur Erzeugung von mit einer Frequenz wiederholten Gasdruckpulsen,
einem Schlagteil (20, 128, 128'), das durch einen Druckgaspuls der Druckgasversorgungseinrichtung beschleunigt werden kann,
einem Prallkörper (22) der von dem beschleunigten Schlagteil (20, 128, 128') getroffen werden und dabei von diesem einen Impuls zur Erzeugung der Druckwelle übernehmen kann,
und einer Einrichtung (44, 58, 60) zum Einstellen eines Druckwertes der Druckgaspulse;
**gekennzeichnet durch** eine Einrichtung (44) automatischen Auswählen aus einer in dem Gerät (10) gespeicherten Tabelle und Einstellen der Zeitdauer der Druckgaspulse und **dadurch, dass** das Gerät (10) für ein Verfahren nach einem der vorstehenden Ansprüche ausgelegt ist.

## Claims

1. A method for setting a pressure gas application duration of an apparatus (10) for treating the human or animal body by a mechanical pressure wave,
said apparatus (10) comprising:
a pressure gas supply device (32) for producing gas pressure pulses repeated with a frequency,
a striking element (20, 128, 128') to be accelerated by a pressure gas pulse of the pressure gas supply device (32),
an impact body (22) to be struck by said accelerated striking element (20, 128, 128') to thereby receive an impulse therefrom for producing the pressure wave,
and a device (44, 58, 60) for setting a pressure value of the pressure gas pulses;
**characterized in that** the apparatus (10) comprises
a device (44) for automatically selecting and setting the time duration of the pressure gas pulses and
**in that**, in the setting method, preset time duration values for respective pressure values are automatically selected and set corresponding to the preset pressure values from a table memorized in the apparatus (10) by the device (44) for automatically selecting and setting the time duration of the pressure gas pulses.

2. The method of claim 1 wherein the time duration values are set such that only one respective collision of the striking element (20, 128, 128') onto the impact body (22) appears per pressure gas pulse.

3. The method of one of the preceding claims wherein the apparatus is constructed such that the striking element (20, 128, 128') displaces air in front thereof along a path of its acceleration and presses said air into a counterpressure chamber (52, 184) in which a counterpressure is caused, consequently, for returning the striking element (20, 128, 128') along said path.

4. The method of one of the preceding claims wherein the time duration values are selected and set exclusively in response to the pressure values set.

5. The method of one of the preceding claims wherein the impact body is suspended elastically so that it can be displaced by an impulse transfer from the striking element (20, 128, 128'), in particular via at least one elastomer ring (56, 150, 150', 151').

6. The method of claim 5 wherein the apparatus (10) is adapted such that values of the displacement in a range of 0.5 mm to 5 mm result.

7. The method of one of the preceding claims wherein the apparatus (10) comprises a security catch (180) for catching the striking element in case of triggering the acceleration thereof while the impact body is dismounted, in particular in the implementation of a tapering (180) at an end to the impact body's side of a tube portion (182) for accelerating the striking element.

8. An apparatus (10) for treating the human or animal body by a mechanical pressure wave, comprising:
a pressure gas supply device (32) for producing gas pressure pulses repeated with a frequency,
a striking element (20, 128, 128') to be accelerated by a pressure gas pulse of the pressure gas supply device,
an impact body (22) to be struck by the accelerated striking element (20, 128, 128') in order to receive an impulse therefrom for producing a pressure wave, and a device (44, 58, 60) for setting a pressure value of the pressure gas pulses,
**characterized by**
a device (44) for automatically selecting from a table memorized in the apparatus (10), and setting the time duration of the pressure gas pulses,
and in that said apparatus (10) is adapted for the method of one of the preceding claims.

## Revendications

1. Procédé de réglage de la durée d'application d'une pression de gaz d'un appareil (10) pour le traitement du corps humain ou animal par une onde de pression mécanique, ledit appareil (10) présentant:
un dispositif d'apport de gaz sous pression (32) permettant de produire des impulsions de pression de gaz répétées à une certaine fréquence,
un élément de frappe (20, 128, 128') susceptible de subir une accélération sous l'effet d'une impulsion de gaz sous pression du dispositif d'apport de gaz sous pression (32),
un corps d'impact (22) pouvant être touché par l'élément de frappe (20, 128, 128') accéléré et pouvant ainsi recevoir une impulsion de la part de celui-ci, produisant ainsi l'onde de pression, et
un dispositif (44, 58, 60) permettant de régler une valeur de pression des impulsions de gaz sous pression;
**caractérisé en ce que**:
l'appareil (10) présente un dispositif (44) permettant de sélectionner et de régler automatiquement la durée des impulsions de gaz sous pression, et
dans le cadre du procédé de réglage, des valeurs de durée prédéfinies peuvent être automatiquement sélectionnées et réglées pour les diverses valeurs de pression à partir d'une table mémorisée dans l'appareil (10) par le dispositif (44) permettant de sélectionner et de régler automatiquement la durée des impulsions de gaz sous pression, conformément aux valeurs de pression réglées.

2. Procédé selon la revendication 1, dans lequel les valeurs de durée sont réglées de manière qu'il ne se produise, pour chaque impulsion de gaz sous pression, qu'une seule percussion de l'élément de frappe (20, 128, 128') sur le corps d'impact (22).

3. Procédé selon l'une quelconque des revendications précédentes, dans lequel l'appareil (10) est conçu de manière que l'élément de frappe (20, 128, 128') repousse le gaz devant lui en suivant la trajectoire de son accélération, en le poussant dans une chambre de contre-pression (52, 184) dans laquelle il se forme ainsi une contre-pression susceptible de renvoyer l'élément de frappe (20, 128, 128') le long de ladite trajectoire.

4. Procédé selon l'une quelconque des revendications précédentes, dans lequel les valeurs de durée sont sélectionnées et réglées exclusivement conformément aux valeurs de pression réglées.

5. Procédé selon l'une quelconque des revendications précédentes, dans lequel le corps d'impact est monté de manière élastique, notamment au moyen d'au moins un anneau élastomère (56, 150, 150', 151') lui permettant d'être dévié sous l'effet de l'élément de frappe (20, 128, 128') lors de la réception de l'impulsion.

6. Procédé selon la revendication 5, dans lequel l'appareil (10) est conçu de manière à produire des valeurs de déviation dans la plage de 0,5 mm à 5 mm.

7. Procédé selon l'une quelconque des revendications précédentes, dans lequel l'appareil (10) présente un dispositif d'interception (180) destiné à intercepter l'élément de frappe en cas de déclenchement de son accélération tandis que le corps d'impact est démonté, notamment sous la forme d'un rétrécissement (180) au niveau d'une extrémité située du côté corps d'impact d'une pièce tubulaire (182) servant à l'accélération de l'élément de frappe.

8. Appareil (10) pour le traitement du corps humain ou animal par une onde de pression mécanique, comportant:
un dispositif d'apport de gaz sous pression (32) permettant de produire des impulsions de pression de gaz répétées à une certaine fréquence,
un élément de frappe (20, 128, 128') susceptible de subir une accélération sous l'effet d'une impulsion de gaz sous pression du dispositif d'apport de gaz sous pression,
un corps d'impact (22) pouvant être touché par l'élément de frappe (20, 128, 128') accéléré et pouvant ainsi recevoir une impulsion de la part de celui-ci, produisant ainsi l'onde de pression, et
un dispositif (44, 58, 60) permettant de régler une valeur de pression des impulsions de gaz sous pression,
**caractérisé par**:
un dispositif (44) permettant de sélectionner, à partir d'une table mémorisée dans l'appareil (10), et de régler automatiquement la durée des impulsions de gaz sous pression, et par le fait que:
l'appareil (10) est conçu pour un procédé selon l'une quelconque des revendications précédentes.
